# EUROPEAN PATENT APPLICATION

(11) **EP 4 706 563 A1**
(43) Date of publication of application: **11.03.2026**
(21) Application number: 25200213.4
(22) Date of filing: 04.09.2025
(51) Int. Cl.: A61B 17/32, F16H 53/06, A61B 17/16

(54) **ACTUATION ASSEMBLY FOR RECIPROCATING SURGICAL TOOL**

(30) Priority: 06.09.2024 US 202463691404 P; 03.09.2025 US 202519317397
(71) Applicant: Arthrex, Inc, Naples, FL 34108-1945 (US)
(72) Inventor: MUSER, Andrew P., Naples, FL, 34108 (US); LOMBARDO, Giuseppe, Naples, FL, 34108 (US); FRITZ, Joseph A., Naples, FL, 34108 (US)
(74) Representative: Lohr, Jöstingmeier & Partner Patent- und Rechtsanwälte mbB

(57) **Abstract**

An actuation assembly for a reciprocating surgical tool includes a reciprocating shaft extending along a longitudinal axis and a rotating body operably connected to the reciprocating shaft. The rotating body forms opposing guide profiles defining an extension profile and a retraction profile of the reciprocating shaft. A rotation of the rotating body relative to the reciprocating shaft causes the opposing guide profiles to operably engage the reciprocating shaft causing the reciprocating shaft to extend responsive to the extension profile and retract responsive to the retraction profile along the longitudinal axis.

## Description

### CROSS-REFERENCE TO RELATED APPLICATIONS

This application claims priority under 35 U.S.C. § 119(e) and the benefit of U.S. Provisional Application No. 63/691,404 entitled ACTUATION ASSEMBLY FOR RECIPROCATING SURGICAL TOOL, filed on September 6, 2024, by Muser, et al., the entire disclosure of which is incorporated herein by reference.

### BACKGROUND

The present disclosure generally relates to a drive mechanism for a rotary power tool. More particularly, the disclosure relates to a reciprocating assembly that converts rotary motion to a reciprocating motion.

### SUMMARY

Surgical accessories and tools may be applied to support a wide variety of procedures and surgical operations. In various operations, surgeons may utilize several tools and related accessories to accomplish procedural steps and techniques. The disclosure provides for a surgical power tool that operates with a variety of actuating assemblies to support diverse applications with a single driver. In particular, the disclosure provides for details related to a reciprocating assembly with a rotary coupling mechanism that may provide for improved drive characteristics, balance, and service life.

In various implementations, the actuation mechanism may include a reciprocating shaft that may extend centrally through a reciprocating assembly. In various implementations, the reciprocating assembly may include a rotating body and at least one guide assembly that operably couples the rotating body to the reciprocating shaft. In operation, the rotating body may be rotated via a drive assembly of the surgical power tool and cause the reciprocating shaft to extend and retract as a result of a pushing and pulling force axially applied to the reciprocating shaft by opposing guide profiles. For example, the opposing guide profiles of the rotating body may include an extension profile and a retraction profile that engage a coupling or bearing assembly to generate the reciprocating motion of the reciprocating shaft.

In some implementations, the disclosure may provide for the reciprocating shaft to engage the rotating body in the form of a rotating disk or wave disk. In such implementations, the wave disk may include opposing sides that define the extension profile and the retraction profile as corresponding crests and troughs disposed on a pushing side and an opposing pulling side of the rotating disk. In operation, a reciprocating trolley in connection with the reciprocating shaft may be forced to extend and retract in a reciprocating motion in response to the engagement of the extension profile on the pushing side pushing a distal follower bearing and the retraction profile on the pulling side engaging a proximal follower bearing. In this configuration, the reciprocating motion of the shaft may be controlled via a radially balanced conversion of a rotation of the wave disk to the reciprocating translation of the reciprocating shaft to improve the operation and longevity of the reciprocating assembly.

In some implementations, the rotating body may correspond to a cylindrical body comprising a shaft bore through which a proximal end portion of the reciprocating shaft extends and retracts. In such implementations, a rotary guide channel may be formed in a radial perimeter wall of the cylindrical body. The rotary guide channel may include opposing sides that form opposing guide profiles. The opposing sides of the rotary guide channel may extend along an extension profile and a retraction profile that may vary in longitudinal position radially about a longitudinal axis of the cylindrical body and aligned with the longitudinal axis of the reciprocating shaft. In this configuration, a roller or bearing may follow the extension profile and the retraction profile about the radial perimeter wall and engage a reciprocating coupling that may extend and retract in response to the travel of the bearing within the rotary guide channel to generate a reciprocating motion of the reciprocating shaft.

These and other features, objects and advantages of the present disclosure will become apparent upon reading the following description thereof together with reference to the accompanying drawings.

### BRIEF DESCRIPTION OF THE DRAWINGS

FIG. 1 is a partially exploded assembly view demonstrating a driver attachment and connection interface of a power tool;
FIG. 2 is a partially exploded assembly view demonstrating subassemblies for a driver attachment for a reciprocating accessory of a power tool;
FIG. 3 is a partially exploded assembly view demonstrating a plurality of components forming a reciprocating assembly for a power tool;
FIG. 4 is a partially exploded assembly view demonstrating a reciprocating trolley for a reciprocating accessory of a power tool;
FIG. 5 is an isometric view and orthographic projections of a rotating guide disk demonstrating a retraction profile and an extension profile that converts a rotary motion to a reciprocating motion of a reciprocating shaft;
FIG. 6 is an isometric view and orthographic projections of a drive shaft assembly for a reciprocating drive assembly for a power tool;
FIG. 7 is a partially exploded assembly view demonstrating a reciprocating assembly including a rotary guide channel formed in a radial perimeter wall; and
FIG. 8 is an exploded assembly view demonstrating further detail of the reciprocating drive assembly demonstrated in FIG. 7.

### DETAILED DESCRIPTION

In the following description, reference is made to the accompanying drawings, which show specific implementations that may be practiced. Wherever possible, the same reference numbers will be used throughout the drawings to refer to the same or like parts. It is to be understood that other implementations may be utilized, and structural and functional changes may be made without departing from the scope of this disclosure.

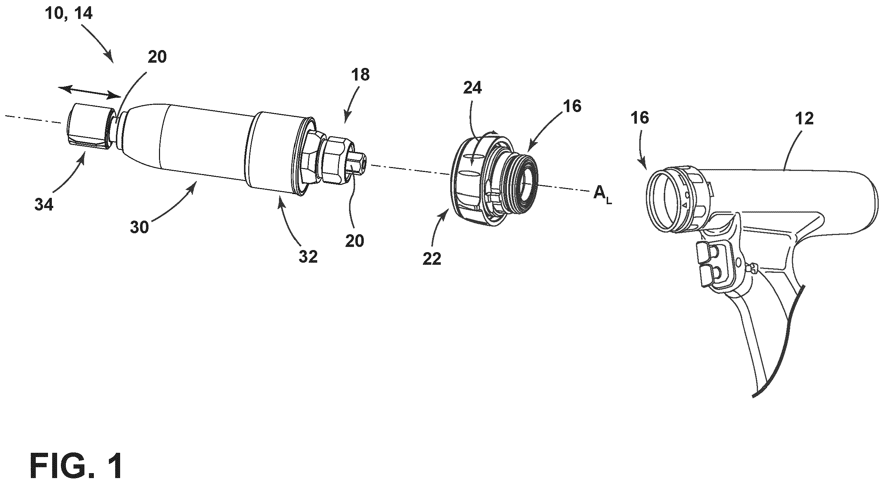

Referring to FIG. 1, the disclosure provides for a reciprocating drive apparatus 10 for a rotary power tool 12. As shown, the drive apparatus 10 may correspond to an interchangeable tool accessory 14 that may be coupled to the rotary power tool via a drive interface 16. For example, the drive interface 16 may comprise a drive shaft 20 that engages a receiving coupler 22 of the rotary power tool 12. In this configuration, the reciprocating drive apparatus 10 may be selectively mounted to the rotary power tool 12 by selectively engaging and disengaging a rotary release collar 24. As further discussed in reference to various examples that follow, the reciprocating drive apparatus 10 may provide for an improved reciprocating assembly 30 with an extended service life and balance for use with various reciprocating accessories (saw blades, files, rasps, chisels, picks, etc.).

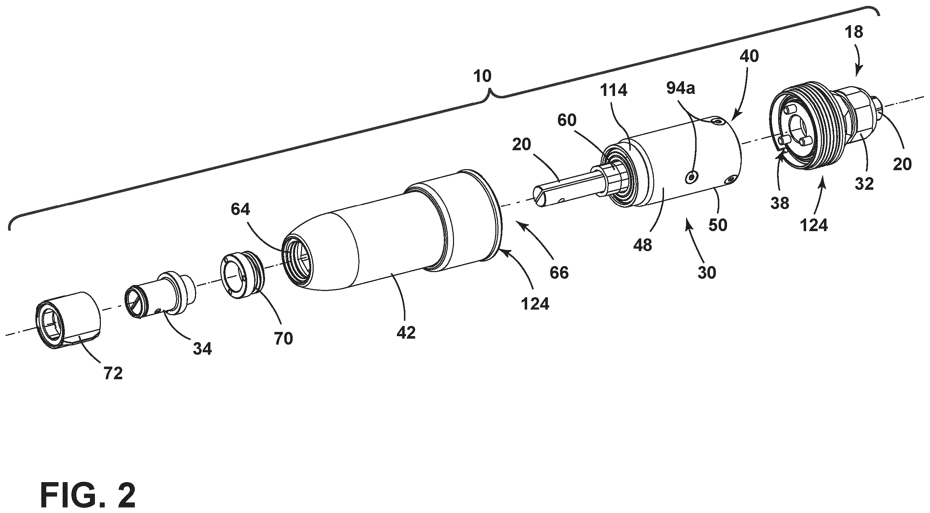

Referring now to FIGS. 1 and 2, the reciprocating drive apparatus 10 may generally comprise a plurality of subassemblies including a drive shaft assembly 32 in connection with a proximal end portion 30a of the reciprocating assembly 30. The reciprocating assembly 30 may comprise a reciprocating shaft 36 that may form a distal end portion 30b of the reciprocating assembly 30. In general, the drive shaft assembly 32 may provide for the connection or drive interface 16 with the rotary power tool 12. In operation, the rotation of the drive shaft 20 may rotationally engage a drive coupling 38 of the drive shaft assembly 32 and rotate a shaft receiver 40 or rotary coupling of the reciprocating assembly 30. The reciprocating assembly 30 may convert the rotation R of the drive shaft 20 and the shaft receiver 40 into a reciprocating motion or translational motion T of the reciprocating shaft 36. In this configuration, the reciprocating assembly 30 may provide for the reciprocating operation of various cutting tools or surgical accessories that may engage the reciprocating shaft 36 via the latch assembly 34.

As best demonstrated in FIG. 2, the reciprocating assembly 30 may be held in engagement with the drive coupling 38 by an outer housing 42. The outer housing 42 may form an enclosure extending about a spindle housing 48 or spindle assembly 50 that rotates within an opening or bore formed by the outer housing 42. The outer housing 42 and the drive shaft assembly 32 may engage the receiving coupler 22 of the power tool 12 and remain in a fixed relationship to the rotary power tool 12 while the drive coupling 38 and shaft receiver 40 rotate within the outer housing 42. In this configuration, an exterior surface formed by the outer housing 42 may serve as a gripping surface or user engagement surface to assist in guiding the operation of a tool or cutting accessory in connection with the reciprocating shaft 36.

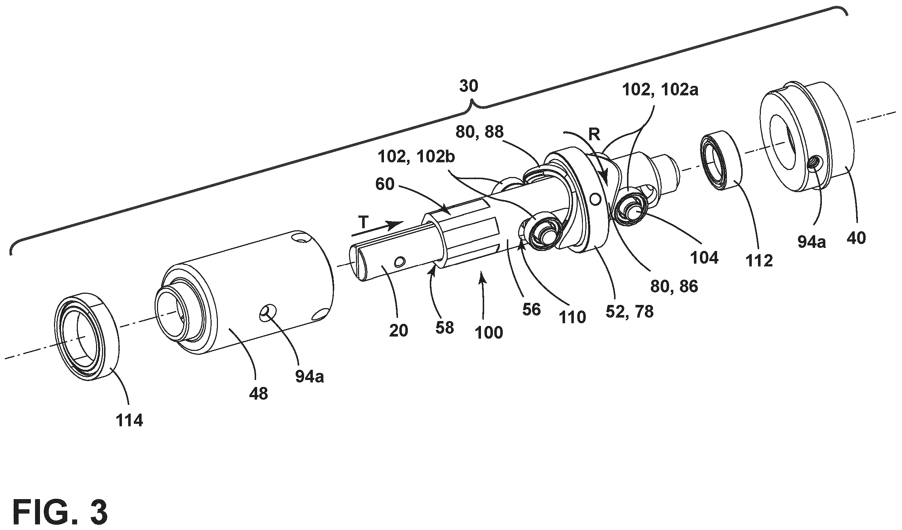

In operation, the rotation of the shaft receiver 40 may result in a corresponding rotation of a spindle housing 48 of a spindle assembly 50. As later discussed in reference to FIGS. 3-5, the rotation of the spindle housing 48 may result in a corresponding rotation of a rotating body 52 disposed within and in connection with the spindle housing 48. As a result of the rotation of the rotating body 52, the reciprocating shaft 36 may be forced to translate relative to the rotating body 52, thereby converting the rotation R of the drive shaft 20 to the translation T of the reciprocating shaft 36. Also disposed within the spindle housing 48, an inner support sleeve 56 may form a reciprocating bore 58 through which the reciprocating shaft 36 extends distally from the spindle assembly 50. The inner support sleeve 56 may form a distal, keyed interface 60 that may engage a complementary keyed socket 62 formed by a distal drive opening 64 of the outer housing 42. In this configuration, the spindle housing 48 may be free to rotate within a spindle bore 66 formed within the outer housing 42 while the inner support sleeve 56 is held in a fixed rotational relationship to the outer housing 42 by the engagement of the distal keyed interface 60 to the complementary keyed socket 62. As further demonstrated in FIG. 2, a distal guide bushing 70 may support the translation T of the reciprocating shaft 36 within a nose cap 72 connected about the distal drive opening 64 of the outer housing 42.

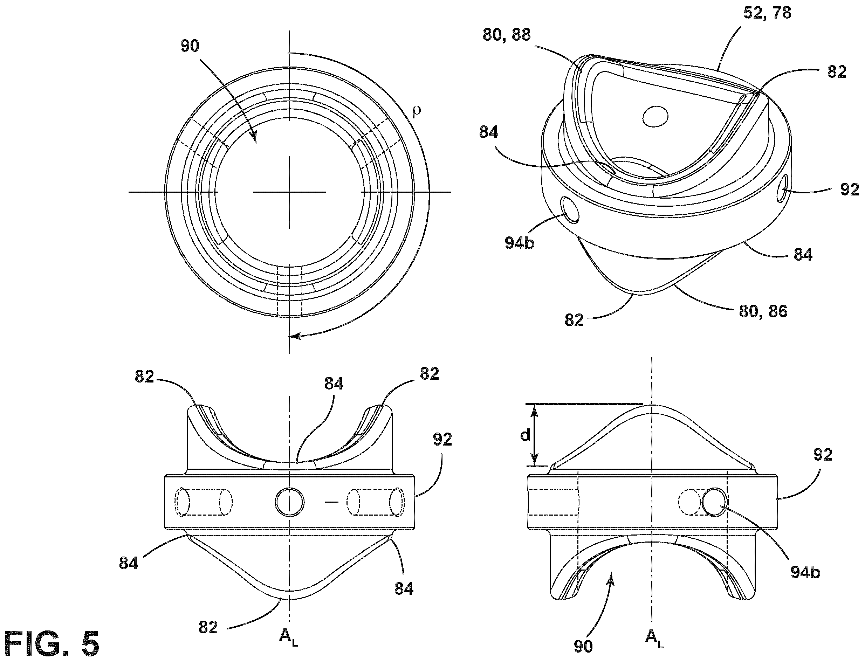

Referring now to FIGS. 3-6, the operation and further detailed features of the reciprocating assembly 30 are discussed, primarily in reference to the operation of the rotating body 52. As best demonstrated in FIGS. 3 and 5, the rotating body 52 may correspond to a disk-like shape or wave disk 78 that may form radially aligned waveforms along opposing guide profiles 80. The opposing guide profiles 80 may be formed by radially periodic crests 82 and troughs 84 formed by a proximal guide surface 86 or pull surface and a distal guide surface 88 or push surface. As demonstrated in FIG. 3, the reciprocating shaft 36 and inner support sleeve 56 may extend along a longitudinal axis A_{L} through a sleeve bore 90 formed transverse to the guide surfaces 86, 88 relative to the longitudinal axis A_{L}. As previously discussed, the spindle housing 48 may be operably connected to the drive coupling 38 via the shaft receiver 40 and be connected to the rotating body 52 via a rotary engagement collar 92. The rotary engagement collar 92 may be interconnected with the inner support sleeve 56 via at least one spindle fastener 94a or pin. In this configuration, the spindle housing 48 may be rotationally coupled to the rotating body 52 based on the engagement of the spindle fastener 94a with a complementary receiving aperture 94b (e.g., a threaded aperture) formed in a radial perimeter of the engagement collar 92. The rotation of the spindle housing 48, responsive to the rotation R of the drive shaft 20, may cause the rotating body 52 or wave disk 78 to rotate about the longitudinal axis A_{L} while maintaining a fixed axial relationship with the spindle housing 48 along the longitudinal axis A_{L}.

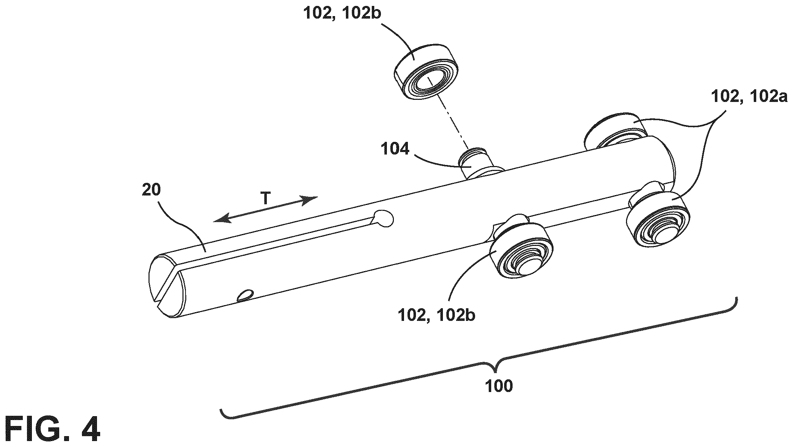

As shown in FIG. 4, the reciprocating shaft 36 may be operably connected to the rotating body 52 and the inner support sleeve 56 via a reciprocating trolley assembly 100. As shown, the reciprocating trolley assembly 100 further includes a plurality of rollers or guides that may engage the opposing guide profiles 80 of the rotating body 52. In the example shown, the rollers are follower bearings 102 and include a proximal follower bearing 102a and a distal follower bearing 102b. In the example shown, a pair of the proximal follower bearings 102a are in connection with a proximal end portion 36a of the reciprocating shaft 36, and a pair of opposing distal follower bearings 102b are in connection with an intermediate portion 36b of the reciprocating shaft 36. As shown in FIG. 3, the proximal follower bearings 102a and distal follower bearings 102b may be positioned on opposing sides of the rotating body 52 and interconnected to the reciprocating shaft 36 by transverse bearing axles 104. In this configuration, the rotation R of the rotating body 52 about the longitudinal axis A_{L} may result in the crests 82 of the opposing guide profiles 80 alternately engaging the proximal follower bearings 102a and the distal follower bearings 102b at a rate corresponding to the rotation R of the spindle housing 48 and angle of the waveforms forming the guide surfaces 86, 88. For example, each full rotation of the spindle housing 48 may result in the reciprocating trolley 100 traversing the crests 82 of each of the opposing guide profiles 80. In the example shown, one full rotation of the spindle housing 48 and the corresponding rotation of the rotating body 52 may cause the reciprocating shaft 36 to extend and retract twice over a reciprocating travel distance d.

Still referring to FIGS. 3-5, as previously discussed, the inner support sleeve 56 may be constrained from rotation within the spindle housing 48 and the spindle assembly 50 as a result of the engagement of the distal keyed interface 60 with the complementary keyed socket 62 of the outer housing 42. Accordingly, the radial orientation of the inner support sleeve 56 may be constrained about the longitudinal axis A_{L} while the spindle housing 48 rotates about the inner support sleeve 56. As best illustrated in FIG. 3, the inner support sleeve 56 may be interposed between the reciprocating shaft 36 and the rotating body 52 extending through the sleeve bore 90. At least one longitudinal guide slot 110 may extend through the inner support sleeve 56 along the longitudinal axis A_{L} providing a reciprocating travel path for the bearing axles 104 to extend and retract over the reciprocating travel distance d responsive to the rotation R of the rotating body 52. In this configuration, the reciprocating trolley 100 may be constrained to translate along the longitudinal axis A_{L} controlling the reciprocating shaft 36 to extend and retract over the travel distance d responsive to the rotation R of the rotating body 52.

As further demonstrated in FIG. 3, the rotation of the spindle housing 48 may be supported on opposing sides of the reciprocating assembly 30 by a proximal support bearing 112 and a distal support bearing 114. In this configuration, the spindle assembly 50 may be proximally supported by the proximal support bearing 112, which may be disposed within the shaft receiver 40. The drive shaft assembly 32 may be distally supported by the distal support bearing 114. The distal support bearing may be interposed between a distal flange 115 of the spindle housing 48 and an interior support bore within the spindle bore 66. In this configuration, the spindle housing 48 may be constrained only to rotate about the longitudinal axis A_{L} and drive the corresponding rotation of the rotating body 52. In response to the rotation of the rotating body 52, the opposing guide profiles 80, including the crests 82 and troughs 84 of the wave disk 78, may alternately engage the follower bearings 102 of the reciprocating trolley 100. In this way, the rotation R of the spindle housing 48 may be converted to the translation T of the reciprocating shaft 36. Additionally, the symmetric structure and nature of the engagement of the follower bearings 102 with the opposing guide profiles 80 of the wave disk 78 or rotating body 52 may provide for a rotationally balanced conversion from the rotation R to the translation T that may not induce imbalance and corresponding reaction forces in the operation.

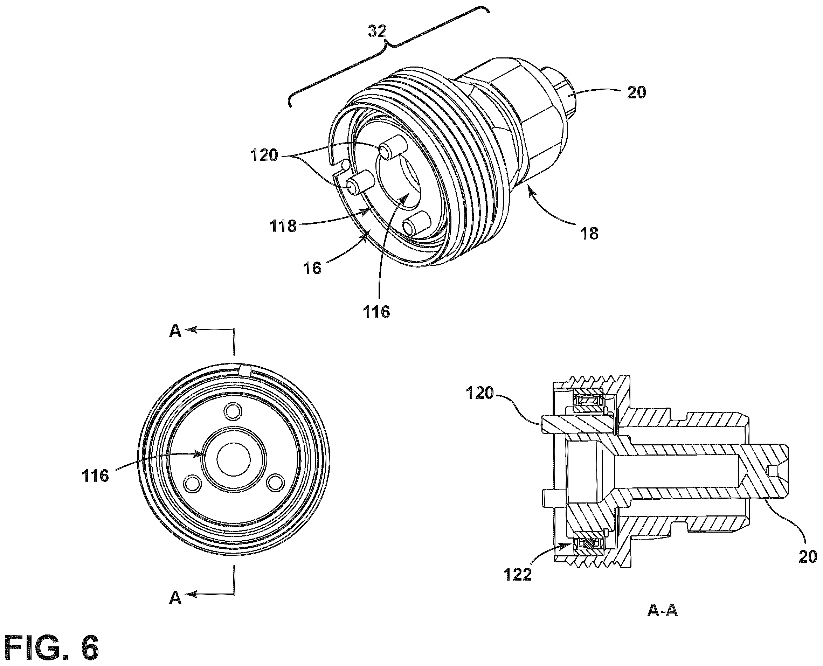

Referring now to FIG. 6, the drive shaft assembly 32 and connection interface 18 are described in further detail. As shown, the drive shaft 20 may extend centrally through a drive shaft bore 116 through the connection interface 18. At the proximal end, the drive shaft assembly 32 may form the drive coupling, which may correspond to a keyed or interlocking shaft configured to engage a corresponding, complementary interface of the rotary power tool 12. A distal end portion of the drive shaft 20 may expand in diameter to form a coupling surface 118 that supports the drive coupling 38. In the example shown, the drive coupling 38 may comprise a plurality of rotary coupling pins 120 that may extend from and be supported by the body of the drive shaft 20 and engage corresponding receiving openings in the shaft receiver 40 of the reciprocating assembly 30. As further demonstrated, the drive shaft 20 may be supported within the drive shaft bore 116 formed by the drive shaft assembly 32 by a drive shaft bearing 122. The drive shaft bearing 122 may be disposed about a distal portion of the drive shaft 20 generally extending about an increased diameter corresponding to the coupling surface 118. In this configuration, the drive shaft 20 may be free to rotate within the connection interface 18 formed by the drive shaft assembly 32 and may further engage the outer housing 42 via complementary engagement surfaces 124 (e.g., a threaded interface) to enclose and retain the reciprocating assembly 30 in mating connection with the drive shaft 20.

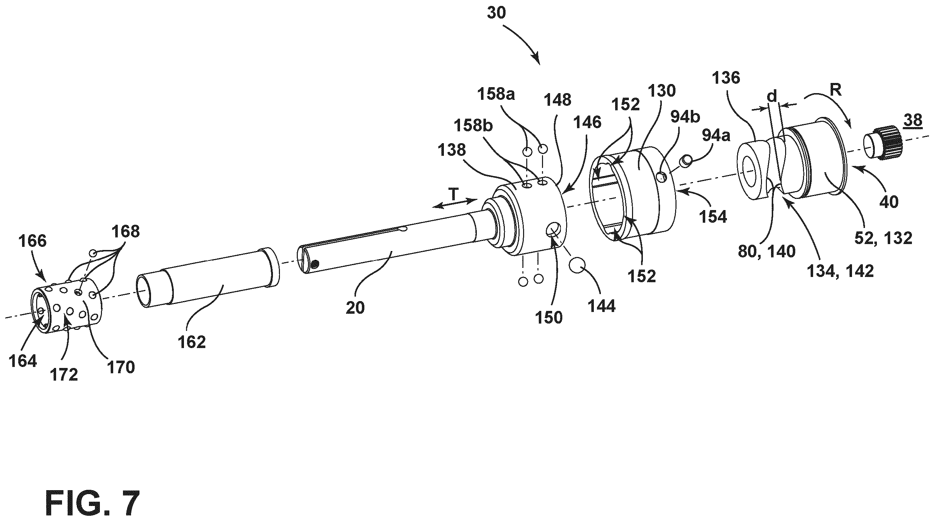

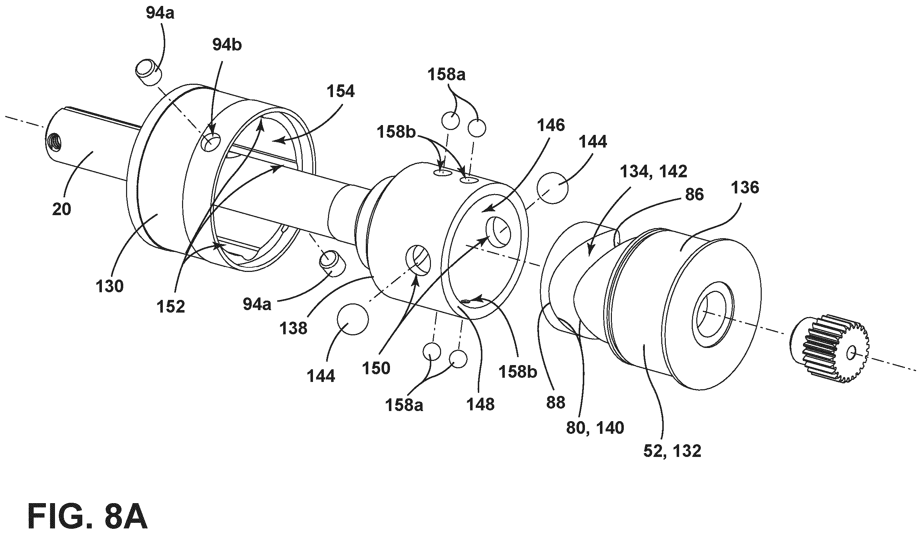

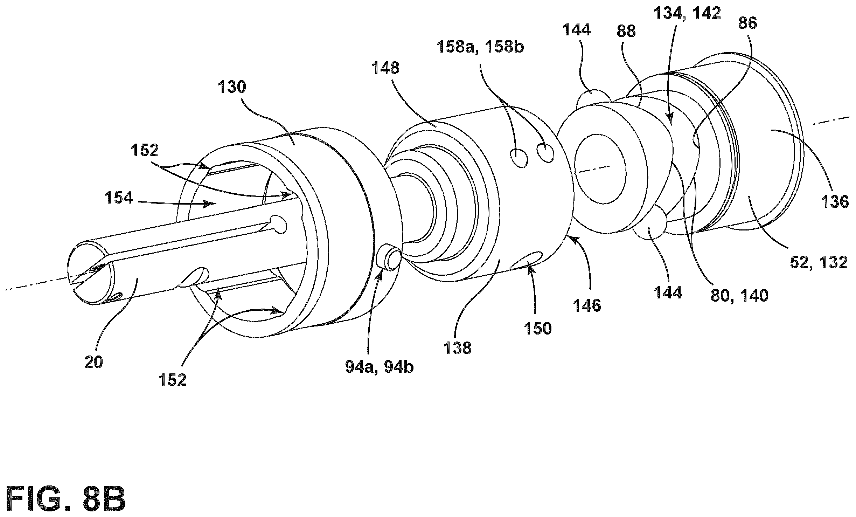

Referring now to FIGS. 7, 8A, and 8B, an additional example of the rotating body 52 of the reciprocating assembly 30 is shown. Similar to the wave disk 78 previously discussed in reference to FIGS. 3-5, the rotating body 52 may comprise a receiver 40 engaged by the drive coupling 38 or a similar coupling assembly to couple the rotation R of the drive shaft 20 to the rotating body 52. Further, the spindle housing 48 of the spindle assembly 50, as previously described in reference to FIG. 2, may be connected to the rotating body 52 via a spindle sleeve 130. As shown, the spindle sleeve 130 may be fixedly coupled to the spindle housing 48 via at least one spindle pin 94a or fastener. In this configuration, the spindle housing 48 may be configured to retain and encase the rotating body 52, allowing the rotating body 52 to rotate therein and drive the reciprocating motion of the drive shaft 20.

Similar to the earlier example described in reference to FIGS. 3-5, the spindle housing 48 may be operably coupled to the spindle sleeve 130 based on the engagement of the spindle pin 94a or fastener with complementary receiving apertures 94b of the sleeve 130 and the housing 48. In this configuration, the conversion of the rotary motion of the rotating body 52 may be converted to the reciprocating motion of the drive shaft 20 within the housing 48 or the reciprocating assembly 30. Accordingly, the reciprocating assembly 30 may be implemented similarly to examples previously discussed. In some implementations, the rotating body 52 shown in the example of FIGS. 7 and 8 may similarly incorporate the opposing guide profiles 80 to convert the rotation R of the rotating body 52 to the translation T of the reciprocating shaft 36. For clarity, the rotating body 52 demonstrated in FIG. 7 may be referred to as a tubular guide member 132 that may form a rotary guide channel 134 about a radial perimeter wall 136.

The operation of the tubular guide member 132 may generally provide for similar operation as previously discussed in reference to the wave disk 78. For example, the reciprocating assembly 30 may rely on a reciprocating coupling 138 to engage the rotating body 52 and convert the rotation R to the translation T of the reciprocating shaft 36. As demonstrated in FIG. 7, the rotary guide channel 134 may comprise opposing side walls 140 extending parallel along a central, cylindrical trough 142. The cylindrical trough 142 may form a periodic waveform that may oscillate longitudinally along a circumferential guide surface extending about the radial perimeter wall 136, such that the opposing side walls 140 provide a similar form and function to the guide surfaces 86, 88 extending along the opposing guide profiles 80 of the wave disk 78. In the example of FIG. 7, the cylindrical trough 142 may receive a rounded surface of at least one spherical guide bearing 144 to guide a reciprocating translational T movement of the reciprocating coupling 138.

In an assembled configuration, a coupling bore 146 extending through and forming the tubular body of the reciprocating coupling 138 may receive the cylindrical profile of the radial perimeter wall 136 formed by the tubular guide member 132. The at least one guide bearing 144 may extend through a coupling side wall 148 of the reciprocating coupling 138 via a coupling aperture 150 and engage the trough 142 operably coupling the rotating body 52 to the to the reciprocating sleeve 130. In the example shown, a plurality of spherical guide bearings 144 may be positioned in opposing coupling apertures 150 formed on opposing sides of the tubular guide member 132 and engage the rotary channel 134. In this configuration, the guide bearings 144 may traverse the cylindrical trough 142 between the opposing side walls 140 in response to the rotation R of the tubular guide member 132. As a result, the guide bearings 144 may force the reciprocating coupling 138 to follow changes in the axial position extending along the longitudinal axis as defined by the wave-like opposing guide profiles 80 formed by the opposing walls 140 of the cylindrical trough 142.

As previously discussed, the reciprocating coupling 138 may be constrained to a translational motion T along the longitudinal axis by the spindle sleeve 130. As shown, the spindle sleeve 130 may form at least one longitudinal trough 152 extending along an interior surface 154 of the spindle sleeve 130. In an assembled configuration with the tubular guide member 132 and the reciprocating coupling 138, the at least one guide bearing 144 may extend from the cylindrical trough 142 through the coupling aperture 150 and into the longitudinal trough 152. Further, the spindle sleeve 130 may be radially and translationally constrained to the spindle housing 48. In this configuration, the reciprocating coupling 138 may translate T along the longitudinal axis A_{L} in response to the guide bearing 144 traversing the opposing guide profiles 80 formed by the opposing side walls 140 of the cylindrical trough 142. The reciprocating coupling 138 may be constrained to the translational motion T by the guide bearings 144 engaging the longitudinal trough 152 formed along the interior surface 154.

In addition to the engagement of the guide bearing(s) 144, the reciprocating coupling 138 may further comprise one or more longitudinal guides 158, which may correspond to reciprocating bearing 158a or raised semicylindrical locating protrusions extending along an exterior surface of the coupling side wall 148. In the example shown, the reciprocating bearings may engage the coupling side wall 148 of the reciprocating coupling 138 via reciprocating apertures 158b. The longitudinal guides 158 may engage corresponding longitudinal troughs 152 formed within the spindle sleeve 130. In the example shown, the reciprocating coupling 138 comprises two opposing longitudinal guides 158 that may be evenly radially spaced with two opposing coupling apertures 150. In this configuration, the guide bearings 144 and the reciprocating bearings 158a may be evenly radially spaced about the spindle sleeve 130 relative to the longitudinal axis A_{L}. Additionally, the reciprocating bearings 158a may engage the coupling sidewall 148 to the corresponding troughs 152 at two or more longitudinally spaced locations along the length of the reciprocating coupling. The longitudinally spaced positions of the reciprocating bearings 158a may provide distributed support along the longitudinal troughs 152 on interior surface 154. Though demonstrated as including two guide bearings 144 and four reciprocating bearings 158a evenly spaced radially about the reciprocating coupling 138, the number, spacing, and utilization of the guide bearings 144 and longitudinal guides 158 may vary. For example, in some cases, additional guide bearings 144 may be implemented evenly spaced about the reciprocating coupling 138 to provide further distributed lateral support. Accordingly, the guide bearings 144 and longitudinal guides 158 may be implemented in various combinations to suit the desired application.

Referring still to FIG. 7, the reciprocating motion of the drive shaft 20 may further be supported by a bearing sleeve 162 that may be enclosed about a proximal end portion. The bearing sleeve 162 may engage an inner bearing wall 164 of a bearing cage 166 along the length of the translational motion T. As shown, the bearing cage 166 may correspond to a tubular sleeve comprising a plurality of bearings 168 that may extend through a sleeve wall 170 from the inner bearing wall 164 to an outer bearing wall 172. In this configuration, the bearing cage 166 may be interposed between the drive shaft 20 and portions of the spindle housing 48, the outer housing 42, and/or additional shells, casings, enclosures that may form fixed portions of the reciprocating assembly 30. In response to the translational motion T of the reciprocating drive shaft, the interposed bearing cage 166 may provide a low friction bearing surface that may support the lateral forces applied to the reciprocating accessories (saw blades, files, rasps, chisels, picks, etc.) connected to the drive shaft 20. In this way, the lateral forces experienced during loaded conditions may be supported by the drive shaft 20, the bearing cage 166, and the portions of the housing(s) 42, 48 limiting the transfer of external forces into the spindle sleeve 130 and reciprocating coupling 138 of the reciprocating assembly 30.

As discussed herein, the disclosure provides for various exemplary features that may be implemented to provide the reciprocating drive apparatus 10 for operation with the rotary power tool 12. Though specific examples are described, it shall be understood that the various features and assemblies provided by the disclosure may be modified and combined to suit various applications. In general, the reciprocating assemblies 30 disclosed may provide for improved operation for implementing reciprocating accessories with rotary power tools in various implementations. In each example, the reciprocating assemblies 30 may provide for radially balanced operation that may improve user satisfaction by limiting reactive forces generated internally by the drive apparatus 10.

According to some aspects of the disclosure, an actuation assembly for a reciprocating surgical tool comprises a reciprocating shaft extending along a longitudinal axis and a rotating body operably connected to the reciprocating shaft, the rotating body forming opposing guide profiles defining an extension profile and a retraction profile of the reciprocating shaft, wherein a rotation of the rotating body relative to the reciprocating shaft causes the opposing guide profiles to operably engage the reciprocating shaft causing the reciprocating shaft to extend responsive to the extension profile and retract responsive to the retraction profile along the longitudinal axis.

According to various aspects, the disclosure may implement one or more of the following features or configurations in various combinations:
- the extension profile and the retraction profile form wave profiles comprise a plurality of crests separated by troughs;
- the extension profile and the retraction profile form wave profiles that are evenly spaced angularly about the longitudinal axis, wherein the extension profile is offset from the retraction profile, such that a crest of the extension profile aligns with a trough of the retraction profile;
- the rotating body comprises a shaft bore operably connected to the reciprocating shaft;
- at least one guide assembly operably coupling the rotating body to the reciprocating shaft, wherein the rotation of the rotating body relative to the reciprocating shaft causes the opposing guide profiles to engage the at least one guide assembly causing the extension and retraction of the reciprocating shaft while constraining a rotation of the reciprocating shaft;
- at least one guide assembly comprises a first guide assembly operably coupled to the reciprocating shaft proximal of the rotating body and a second guide assembly operably coupled to the reciprocating shaft distal of the rotating body;
- the rotating body comprises a rotating disk forming the shaft bore and the rotating disk is interposed between the first guide assembly and the second guide assembly, wherein the rotating disk engages the first guide assembly and the second guide assembly adjusting an axial position of the reciprocating shaft along the longitudinal axis;
- an angular position of the rotating disk relative to the reciprocating shaft controls the axial position of the reciprocating shaft;
- rotating disk comprises a first guide profile that engages the first guide assembly in response to a rotation of a driveshaft operably coupled to the rotating disk;
- the rotating body includes a cylindrical body comprising a bore that receives the reciprocating shaft;
- at least one guide assembly comprises at least one bearing that follows a rotary guide channel formed in a radial perimeter wall of the rotating body;
- the rotary guide channel comprises opposing sides spaced along the longitudinal axis and forming the opposing guide profiles;
- a proximal wall of the opposing sides defines the extension profile and a distal wall of the opposing sides defines the retraction profile;
- at least one guide assembly further comprises a reciprocating body interconnecting the rotating body to the reciprocating shaft, wherein the reciprocating body forms a collar comprising at least one receiving aperture that receives the at least one bearing;
- an axial guide sleeve comprising at least one longitudinal guide channel formed in a reciprocating guide bore;
- the longitudinal guide channel receives the at least one bearing extending from the rotary guide channel and through the at least one receiving aperture; and/or
- the reciprocating body comprises at least one translational locator angularly space from the at least one receiving aperture, wherein the translational locator engages the at least one guide channel constraining a motion of the reciprocating body to extend along the longitudinal axis.

According to another aspect of the disclosure, an actuation mechanism for a reciprocating surgical tool comprises a reciprocating shaft extending along a longitudinal axis; a first guide assembly operably coupled transverse to the longitudinal axis; a second guide assembly operably coupled transverse to the longitudinal axis distal of the first guide assembly; and a rotating disk comprising a disk bore that receives the reciprocating shaft interposed between the first guide assembly and the second guide assembly, wherein the rotating disk engages the first guide assembly and the second guide assembly, adjusting an axial position of the reciprocating shaft along the longitudinal axis.

According to various aspects, the disclosure may implement one or more of the following features or configurations in various combinations:
- a guide sleeve interposed between the reciprocating shaft and the rotating disk;
- the guide sleeve comprises a tubular body including at least one slot extending along the longitudinal axis;
- the reciprocating shaft, the first guide assembly, and the second guide assembly form a reciprocating trolley constrained to translate axially along the longitudinal axis along the at least one slot in response to a rotation of the rotating disk;
- the guide sleeve is operably coupled to an outer housing having a fixed orientation relative to a rotary drive tool of the rotary driveshaft;
- the rotating disk is coupled to the rotary driveshaft via a tubular coupling, and the rotating disk is disposed in a coupling bore of the tubular coupling;
- the tubular coupling forms a spindle housing enclosed about the rotating disk, the first guide assembly, and the second guide assembly;
- at least one of the first guide assembly and the second guide assembly each comprise a bearing assembly connected transverse to the longitudinal axis; and/or
- the bearing assembly comprises an axle extending in transverse connection with the reciprocating shaft and bearings in connection with the axle on opposing sides of the reciprocating shaft.

According to yet another aspect of the disclosure, an actuation assembly for a reciprocating surgical tool comprises a reciprocating shaft extending along a longitudinal axis; a rotating body comprising a shaft bore operably connected to the reciprocating shaft, the rotating body forming opposing guide profiles defining an extension profile and a retraction profile of the reciprocating shaft; and at least one guide assembly operably coupling the rotating body to the reciprocating shaft, wherein a rotation of the rotating body relative to the reciprocating shaft causes the opposing guide profiles to engage the at least one guide assembly causing the reciprocating shaft to extend responsive to the extension profile and retract responsive to the retraction profile along the longitudinal axis.

It will be understood that any described processes or steps within described processes may be combined with other disclosed processes or steps to form structures within the scope of the present device. The exemplary structures and processes disclosed herein are for illustrative purposes and are not to be construed as limiting.

It is also to be understood that variations and modifications can be made on the aforementioned structures and methods without departing from the concepts of the present device, and further it is to be understood that such concepts are intended to be covered by the following claims unless these claims by their language expressly state otherwise.

The above description is considered that of the illustrated embodiments only. Modifications of the device will occur to those skilled in the art and to those who make or use the device. Therefore, it is understood that the embodiments shown in the drawings and described above are merely for illustrative purposes and not intended to limit the scope of the device, which is defined by the following claims as interpreted according to the principles of patent law, including the Doctrine of Equivalents

## Claims

1. An actuation assembly for a reciprocating surgical tool comprising:
a reciprocating shaft (36) extending along a longitudinal axis (A_{L}); and
a rotating body (52) operably coupled to the reciprocating shaft (36), the rotating body (52) forming opposing guide profiles (80) defining an extension profile and a retraction profile of the reciprocating shaft (36), wherein a rotation of the rotating body (52) relative to the reciprocating shaft (36) causes the opposing guide profiles (80) to operably engage the reciprocating shaft (36) causing the reciprocating shaft (36) to extend responsive to the extension profile and retract responsive to the retraction profile along the longitudinal axis (A_{L}).

2. The actuation assembly according to claim 1, wherein the extension profile and the retraction profile form wave profiles comprising a plurality of crests (82) separated by troughs (84).

3. The actuation assembly according to claim 1, wherein the extension profile and the retraction profile extend about a circumferential surface of the rotating body (52).

4. The actuation assembly according to claim 3, wherein the extension profile and the retraction profile extend about a circumferential surface to form a lateral trough and extend along a cyclic gradual curve.

5. The actuation assembly according to any one of claims 1-4, wherein the extension profile and the retraction profile form wave profiles that are evenly spaced angularly about the longitudinal axis (A_{L}), wherein the extension profile is offset from the retraction profile, such that a crest (82) of the extension profile aligns with a trough (84) of the retraction profile.

6. The actuation assembly according to any one of claims 1-5, wherein the rotating body (52) comprises a shaft bore (116) operably connected to the reciprocating shaft (36).

7. The actuation assembly according to claim 6, further comprising:
at least one guide assembly operably coupling the rotating body (52) to the reciprocating shaft (36), wherein the rotation of the rotating body (52) relative to the reciprocating shaft (36) causes the opposing guide profiles (80) to engage the at least one guide assembly causing the extension and retraction of the reciprocating shaft (36) while constraining a rotation of the reciprocating shaft (36).

8. The actuation assembly according to claim 7, wherein the at least one guide assembly comprises:
a first guide assembly operably coupled to the reciprocating shaft (36) proximal of the rotating body (52); and
a second guide assembly operably coupled to the reciprocating shaft (36) distal of the rotating body (52).

9. The actuation assembly according to any one of claims 1-8, further comprising:
at least one guide assembly that comprises at least one bearing that follows a rotary guide channel (134) formed in a radial perimeter wall (136) of the rotating body (52).

10. The actuation assembly according to claim 9, wherein the rotary guide channel (134) comprises opposing sides (140) spaced along the longitudinal axis (A_{L}) and forming the opposing guide profiles (80).

11. The actuation assembly according to claim 10, wherein a proximal wall of the opposing sides (140) defines the extension profile and a distal wall of the opposing sides (140) defines the retraction profile.

12. The actuation assembly according to claim 9, wherein the at least one guide assembly further comprises:
a reciprocating body interconnecting the rotating body (52) to the reciprocating shaft (36), wherein the reciprocating body forms a collar (92) comprising at least one receiving aperture that receives the at least one bearing.

13. The actuation assembly according to claim 12, further comprising:
an axial guide sleeve comprising at least one longitudinal guide channel formed in a reciprocating guide bore.

14. The actuation assembly according to claim 13, wherein the longitudinal guide channel receives the at least one bearing extending from the rotary guide channel (134) and through the at least one receiving aperture.

15. The actuation assembly according to claim 14, wherein the reciprocating body comprises at least one translational locator angularly spaced from the at least one receiving aperture, wherein the translational locator engages the at least one guide channel constraining a motion of the reciprocating body to extend along the longitudinal axis (A_{L}).
